(19) Europäisches Patentamt
European Patent Office
Office européen des brevets

(11) Veröffentlichungsnummer : **0 112 586 B1**

# (12) EUROPÄISCHE PATENTSCHRIFT

(45) Veröffentlichungstag der Patentschrift : **15.03.89**

(21) Anmeldenummer : **83113173.5**

(22) Anmeldetag : **28.12.83**

(51) Int. Cl.[4] : **C 07 C 39/12**, C 07 C 37/20, A 61 K 31/05, C 07 C 37/055, C 07 C 39/367, A 61 K 31/055

## (54) Substituierte 4'-Hydroxy-alpha-äthylbenzhydrole zur Verwendung als Arnzeimittel.

(30) Priorität : **28.12.82 HU 418682**

(43) Veröffentlichungstag der Anmeldung : **04.07.84 Patentblatt 84/27**

(45) Bekanntmachung des Hinweises auf die Patenterteilung : **15.03.89 Patentblatt 89/11**

(84) Benannte Vertragsstaaten : **AT BE CH DE FR GB IT LI LU NL SE**

(56) Entgegenhaltungen :
**EP–A– 0 112 588**
**DE–A– 2 438 399**
**ARZNEIMITTELFORSCHUNG/DRUG RESEARCH, Band 28, Teil I, Heft 4, April 1978, pp. 673-677; DE, M. LEDNICZKY u.a.: "Metabolism of 14C-3-trifluoromethyl-alpha-ethyl-benzhydrol in rats"**
**CHEMICAL ABSTRACTS, Band 92, Nr. 7, 18. Februar 1980, p. 158, ref.no. 52927b; Columbus, Ohio, US, I. SZINAI u.a.: "Metabolism of two benzhydrol-compounds in rats"**
**HOUBEN-WEYL/METHODEN DER ORGANISCHEN CHEMIE, 4. Auflage, Band XIII, Teil 1: "Metallorganische Verbindungen, 1970; Georg Thieme Verlag, Stuttgart, DE, pp. 87-253: U. SCHÖLLKOPF: "Methoden zur Herstellung und Umwandlung von lithium-organischen Verbindungen"**
**pp. 255-725: H.F. EBEL u.a.: "Methoden zur Herstellung und Umwandlung von alkalimetall-organischen Verbindungen (ausser denen des Lithiums)"**

(73) Patentinhaber : **RICHTER GEDEON VEGYESZETI GYAR R.T.**
**Gyömröi ut 19-21**
**H-1475 Budapest X (HU)**

(72) Erfinder : **Tòth, Edit, Dipl.-Ing.**
**Szabolcska u. 7**
**H-1114 Budapest (HU)**
Erfinder : **Törley, Jòzsef, Dipl.-Ing.**
**Katona J. u. 41**
**H-1137 Budapest (HU)**
Erfinder : **Fekete, György, Dr.**
**Széher u. 62**
**H-1021 Budapest (HU)**
Erfinder : **Szporny, Lászlò, Dr.**
**Szabolcska u. 7**
**H-1114 Budapest (HU)**
Erfinder : **Vereczkey, Lászlò, Dr.**
**Lajos u. 109**
**H-1036 Budapest (HU)**
Erfinder : **Pálosi, Eva, Dr.**
**Vend u. 21**
**H-1025 Budapest (HU)**
Erfinder : **Klebovich, Imre, Dr.**
**Karvaly u. 4**
**H-1125 Budapest (HU)**
Erfinder : **Vittay, Pál, Dr.**
**Jòzsef krt. 14**
**H-1085 Budapest (HU)**
Erfinder : **Görög, Sándor, Dr. Dipl.-Chem.**
**Vajda P. u. 43**
**H-1089 Budapest (HU)**
Erfinder : **Hajdu, István, Dipl.-Chem.**
**Tátra tér B/4**
**H-1205 Budapest (HU)**

(74) Vertreter : **Beszédes, Stephan G., Dr.**
**Münchener Strasse 80a Postfach 1168**
**D-8060 Dachau (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft substituierte 4'-Hydroxy-α-äthylbenzhydrole zur Verwendung als Arzneimittel zur Behandlung von akuten Alkoholvergiftungen.

Das 3-Trifluormethyl-4'-hydroxy-α-äthylbenzhydrol ist als ein Metabolit des das Enzymsystem der Leber induzierenden 3-Trifluormethyl-α-äthylbenzhydrols in Arzneimittelforschung 28 (I), Heft 4, 1978, S. 673-677 beschrieben, wobei angegeben ist, daß diese Verbindung eine selektive induktive Wirkung auf das hepatische Arzneimittel metabolisierende Enzymsystem ausübt und keine andere pharmakologische Wirkung zeigt.

Der Metabolismus des ähnlich wirkenden 2,5-Dimethyl-α-äthylbenzhydrols wurde von Szinai et al. (Proc. 19th Hung. Ann. Meet. Biochem., 1979, Seiten 105-106) untersucht. Die Autoren teilten mit, daß aus dem 2,5-Dimethyl-α-äthylbenzhydrol im Laufe der Biotransformation Hydroxylgruppen enthaltende Verbindungen entstehen. Auch diese Literaturstelle vermittelt nicht den geringsten Hinweis auf die Möglichkeit, daß die erfindungsgemäß eingesetzten 4'-Hydroxy-α-ethylbenzhydrole zur Behandlung von akuten Alkoholvergiftungen geeignet sind.

Es wurde nun gefunden, daß die Verbindungen 3-Trifluormethyl- und 2,5-Dimethyl-4'-hydroxy-α-äthylbenzhydrol zur Behandlung akuter Äthanolintoxikation geeignet und für diesen Zweck ausgedehnt verwendbar sind. Die akute Alkoholintoxikation ist vor allem durch Euphorie, allgemeine Stimuliertheit, Ataxie, Somnolenz und paralytische Zustände charakterisiert. Die Gefahren dieses toxischen, krankhaften Zustandes sind bekannt und nicht zu vernachlässigen. Die intoxierte Person gefährdet ihre Umgebung (Trunkenheit am Steuer) und sich selbst.

Die DE-A-24 38 399 betrifft α-substituierte Benzhydrol-Derivate, die sich von den erfindungsgemäß zu verwendenden 3-Trifluormethyl- und 2,5-Dimethyl-4'-hydroxy-α-ethylbenzhydrolen durch das Fehlen einer OH-Gruppe in der p-Stellung im rechten Benzolring unterscheiden. Die bekannten Benzhydrole wirken induzierend auf das die körperfremden Stoffe metabolisierende mikrosomale Enzymsystem der Leber, wodurch die metabolisierende Aktivität der Leber für xenobiotische Stoffe, beispielsweise Arzneimittel und Steroide, abgesenkt werden kann und damit die Zeitdauer der Anwesenheit und Wirksamkeit dieser xenobiotischen Stoffe im Organismus verlängert wird. Auf eine Behandlung von akuten Alkoholvergiftungen findet man in dieser Literaturstelle nicht den geringsten Hinweis.

Die in dieser DE-A beschriebenen α-substituierten Benzhydrol-Derivate können in der Weise hergestellt werden, daß ein Propiophenon mit einem Phenylmagnesiumhalogenid umgesetzt wird oder eine Grignardverbindung mit Propiophenon zur Umsetzung gebracht wird.

Die akute Alkoholintoxikation ist für den ischämischen Gehirninfarkt ein bedeutender Risikofaktor (Hillbom, M. u. a. : Lancet 2, 1181/1978/ ; Stroke 12, 422/1981/). Für den alkoholintoxierten Zustand gibt es kein geeignetes Antidotum. Die durch Alkohol ausgelöste lokomotorische Hyperaktivität wird an Mäusen durch α-Methyl-para-thyrosin in einem Dosisbereich normalisiert, in dem die Verbindung die spontane lokomotorische Aktivität der Tiere beeinträchtigt (Carlsson, A. u. a. : Psychopharm. 26, 307, 1972). Die narkotisierende Wirkung des Alkohols wird durch Stimulantien (Coffein, Amphetamin) verringert, die motorische Inkoordination (Ataxie) jedoch gleichzeitig prolongiert (Wallagsen, H. u. a. : Actions of alcohol, Amsterdam, Elsevier, 1970 ; Rech; R.H. u. a. : Ann. N.Y. Acad. Sci. 28, 426, 1976 ; Todzy, I. u. a. : Psychopharm. 59, 143, 1978). Die alkoholische Intoxikation, die Narkose wird durch L-Cystein verkürzt (Sprince, H. u. a. : Agents and Actions, 4, 125, 1974 ; Nagasawa, H.T. u. a. : Life Sci., 17, 707, 1975) ; L-Cystein wurde zu den folgenden Untersuchungen über Schlaf unter Alkoholeinfluß als Referenzsubstanz verwendet.

Zur Messung der Änderung der durch Alkohol hervorgerufenen Narkosedauer wurden 16 Stunden lang ausgehungerte, 160-180 g schwere Hann.-Wistar-Ratten beiderlei Geschlechts verwendet. Jede Gruppe bestand aus 10 Tieren, die mit entsprechenden Dosen der Versuchsverbindungen oral behandelt wurden. Eine Stunde nach der Behandlung wurde den Tieren in einer Dosis von 3,5 g/kg intraperitoneal Äthanol appliziert. Die Schlafdauer der Tiere wurde vom Ausfall des Aufrichtreflexes (Righting Reflexes) bis zur spontanen Korrektur der Körperhaltung gemessen. Für jede Gruppe wurde die durchschnittliche Schlafzeit, die Standardabweichung und das Ergebnis in Prozent der Kontrolle berechnet. Die Kontrolle erhielt Placebo und ebenfalls 3,5 g/kg Alkohol. Die Ergebnisse sind in der folgenden Tabelle 1 angegeben, in der $\bar{x} \pm$ S.E. Durchschnittswert $\pm$ Standardabweichung bedeutet. A = 3-Trifluormethyl-4'-hydroxy-α-äthylbenzhydrol Schlafdauer der Kontrolle : 87,4 $\pm$ 6,91 ($\bar{x} \pm$ S.E.) Minuten

Tabelle 1

| Behandlung | Dosis | Schlafdauer (in % der Kontrolle $\pm$ S.E |
|---|---|---|
| Verbindung A | 40,0 | 53 $\pm$5,8 |
| | 80,0 | 62 $\pm$5,1 |
| L-Cystein | 500,0 | 63$\pm$ 4,2 |
| | 1000,0 | 66 $\pm$5,9 |
| Kontrolle | - | 100 $\pm$7,9 |

Die durch die Verbindung A in einer Dosis von 40,0 mg/kg auf die Dauer des Narkoseschlafes ausgeübte Wirkung wurde auch als Funktion der zwischen Behandlung und Alkoholgabe verstrichenen Zeit untersucht, wobei die Versuchsweise die gleiche war wie oben. Die Ergebnisse sind in der Tabelle 2 festgehalten.

Tabelle 2

Wirkung der Verbindung A auf die Schlafdauer bei unterschiedlichen Vorbehandlungszeiten

| Zeit zwischen Behandlung und Alkoholgabe (Stunden) | Schlafdauer Kontrolle | Schlafdauer behandelt | % |
|---|---|---|---|
| 0,5 | 87,2 ±6,23 | 54,1 ±3,49 | -38 |
| 1,0 | 85,1 ±4,91 | 45,1 ±2,30 | -47 |
| 2,0 | 85,5 ±9,97 | 29,9 ±5,08 | -65 |
| 3,0 | 83,4 ±8,46 | 75,1 ±4,51 | -10 |

Aus den Ergebnissen ist ersichtlich, daß die Verbindung die Dauer des Alkoholschlafes bedeutend verkürzt, daß ihre Wirkung besser ist als die des in 10facher Dosis verwendeten L-Cysteins und daß das Wirkungsmaximum zwei Stunden nach der Verabreichung eintritt.

Die akute Toxizität der Verbindung wurde an 10 Ratten beiderlei Geschlechts und eines Gewichtes von 160-180 g untersucht. Den Tieren wurde die zu untersuchende Substanz oral in einer einmaligen Dosis von 2 000 mg/kg appliziert. Innerhalb der 14tägigen Beobachtungszeit verendeten 40 % der Tiere, d. h. der $LD_{50}$-Wert der Verbindung liegt über 2 000 mg/kg, der Toxizitätswert ist günstig.

Durch Untersuchungen auf die im folgenden angegebene Weise wurde festgestellt, daß die Verbindung auch in einer Dosis von 120 mg/kg keinerlei sonstige Wirkung auf das Zentralnervensystem ausübt: Elektroschock (Swinyard, E.A., Brown, W.C., Goodman, L.S. : J. Pharmacol. Exp. Ther. 106, 319/1952), Metrazolkrampf (Everett, G.M., Richards, R.K. : J. Pharmacol. Exp. Ther. 81, 402/1944/), Thiosemicarbazidkrampf (Da Vanzo, J.P., Greig, M.E., Cormin, M.A. : Amer. J. Physiol. 201, 833/1961/), Strychninkrampf (Kerley, T.L., Richards, A.G., Begley, R.W., Abreu, B.B., Wesver, L.C. : J. Pharmacol. Exp. Ther. 132, 360/1961/), Nikotinkrampf (Stone, C.A., Mecklenburg, K.L., Torhans, M.L. : Arch. Int. Pharmacodyn. 117, 419/1958/), Drehstab (Kinnard, W.J., Carr, C.J. : J. Pharmacol. Exp. Ther. 121, 354/1957/), Physostigminletalität (Nose, T. und Kojima, M. : Europ. J. Pharmacol. 10, 83/1970/), yohimbinpotenzierende Wirkung (Quinton, R.M. : Brit. J. Pharmacol. 21, 51/1963/), schmerzstillende Wirkung (Bianchi, C., Franceschini, J. : Brit. J. Pharm. Chemother. 9, 280/1954/).

Die Verbindungen werden erfindungsgemäß zu Arzneimittelpräparaten zubereitet. Die Präparate können oral, rektal und/oder parenteral verabreicht werden. Zur oralen Darreichung können Tabletten, Dragees oder Kapseln hergestellt werden. Bei der Herstellung von oral verabreichbaren Formen werden als Streckmittel zum Beispiel Milchzucker oder Stärke verwendet. Als Binde- oder Granuliermittel kommen zum Beispiel, Gelatine, Carboxymethylcellulose-natrium, Methylcellulose, Polyvinylpyrrolidon oder Stärkekleister in Betracht. Als Sprengmittel werden in erster Linie Kartoffelstärke oder mikrokristalline Cellulose zugesetzt, jedoch können beispielsweise auch Ultraamylopektin oder Formaldehydcasein verwendet werden. Als Antihaftmittel und Gleitmittel kommen z. B. Talk, kolloidale Kieselsäure, Stearin, Ca- und Mg-Stearat in Frage.

Die Tabletten können zum Beispiel durch Naßgranulieren und anschließendes Pressen hergestellt werden. Das Gemisch aus Wirkstoff und Füllstoffen sowie gegebenenfalls ein Teil des Sprengmittels werden mit der wäßrigen, alkoholischen oder wäßrig-alkoholischen Lösung der Bindemittel in einer geeigneten Vorrichtung granuliert, und das Granulat wird getrocknet. Zu dem trockenen Granulat werden der Rest des Sprengmittels, ferner die Antihaftmittel und das Gleitmittel gegeben, und das Gemisch wird zu Tabletten gepreßt. Gegebenenfalls können die Tabletten zur Erleichterung der Dosierung mit Teilungsmarkierungen versehen werden. Die Tabletten können auch unmittelbar durch Pressen eines Gemisches aus dem Wirkstoff und geeigneten Hilfsstoffen hergestellt werden. Die Tabletten können gewünschtenfalls unter Verwendung der in der Arzneimittelherstellung üblichen Schutz-, Geschmacks- und Farbstoffe, zum Beispiel Zucker, Cellulose derivate, wie Methyl- oder Äthylcellulose oder Carboxymethylcellulose-Na, Polyvinylpyrrolidon, Calciumphosphat, Calciumcarbonat, Lebensmittelfarbstoffe, Lebensmittelfarblacke, Aromastoffe und Eisenoxydpigmente, auf die übliche Weise dragiert werden.

Zur Herstellung von Kapseln wird das Gemisch aus Wirkstoff und Hilfsstoffen in Kapseln gefüllt.

Zur rektalen Anwendung werden Suppositorien hergestellt. Diese enthalten außer dem Wirkstoff eine Trägermasse, das sog. Adeps pro suppositori. Als dieses kommen Pflanzenfette, zum Beispiel gehärtete

Pflanzenöle, die Triglyceride von Fettsäuren mit 12-18 Kohlenstoffatomen, vorzugsweise die Trägerstoffe der Markenbezeichnung Witepsol® in Betracht. Der Wirkstoff wird in der geschmolzenen Trägermasse homogen verteilt und das erhaltene Gemisch zu Suppositorien vergossen.

Zur parenteralen Verabreichung werden Präparate in Injektionslösungsform hergestellt. Dazu werden die Wirkstoffe, gegebenenfalls in Gegenwart von Lösungsvermittlern, wie Polyoxyäthylensorbitanmonolaurat, -monooleat oder -monostearat (Tween 20®, Tween 60®, Tween 80®), in destilliertem Wasser und/oder unterschiedlichen organischen Lösungsmitteln, zum Beispiel Glykoläthern, gelöst. Die Injektionslösungen können ferner unterschiedliche Hilfsstoffe, zum Beispiel Konservierungsmittel, wie Benzylalkohol, p-Oxybenzoesäuremethyl- oder -propylester, Benzalkoniumchlorid oder Phenylmercuriborat, Antioxydantien, wie Ascorbinsäure, Tocopherol oder Natriumpyrosulfat, zum Binden von Metallspuren Komplexbildner, z. B. Äthylendiamin-tetraessigsäure, Puffersubstanzen und Stoffe zum Einstellen des pH-Wertes sowie gegebenenfalls Lokalanästhetika, wie Lidocain, enthalten. Die Injektionslösungen werden vor dem Füllen in die Ampullen filtriert und nach dem Füllen sterilisiert.

Die Tagesdosis beträgt, abhängend vom Zustand des Kranken, 0,1-300 mg/kg, vorzugsweise 2,0-160 mg/kg, und wird zweckmäßig in kleineren Einzeldosen verabreicht.

Gegenstand der Erfindung sind daher substituierte 4'-Hydroxy-α-äthylbenzhydrole der allgemeinen Formel

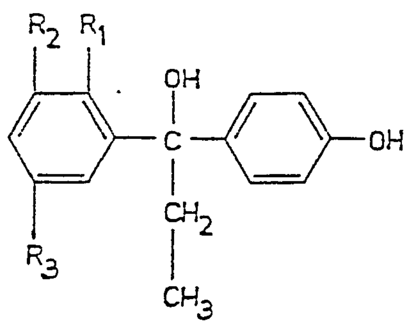

0,

worin

$R_1$ und $R_3$ für Methylreste stehen und
$R_2$ Wasserstoff bedeutet, oder
$R_1$ und $R_3$ für Wasserstoff stehen und
$R_2$ einen Trifluormethylrest bedeutet,

zur Verwendung als Arzneimittel zur Behandlung von akuten Alkoholvergiftungen.

Die obigen substituierten 4'-Hydroxy-α-äthylbenzhydrole können in an sich bekannter Weise dadurch hergestellt werden, daß 4-Hydroxypropiophenon mit eine substituierte Phenylgruppe aufweisenden metallorganischen Verbindungen der allgemeinen Formel

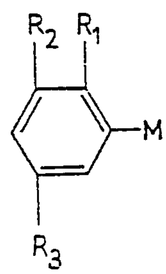

(I)

worin

M für ein Alkalimetallatom, vorzugsweise Lithium-, Natrium- oder Kaliumatom, oder einen Rest der allgemeinen Formel MgX mit X = Halogen steht und
$R_1$, $R_2$ und $R_3$ wie oben festgelegt sind,

umgesetzt und die erhaltenen metallorganischen Verbindungen in üblicher Weise zersetzt werden.

Die Ausgangsstoffe sind bekannt beziehungsweise können nach literaturbekannten Verfahren erhalten werden. Die Verbindungen der allgemeinen Formel (I) können zum Beispiel hergestellt werden, indem man aus einem entsprechend substituierten Arylhalogenid in literaturbekannter Weise ein Grignard-Reagens bereitet (s. z. B. M.S. Kharash u. a. : Grignard reactions of nonmetallic substances, Ed. Prentice-Hall. Inc. 1954, S. 5-90) ; die alkalimetallorganischen Verbindungen sind zum Beispiel nach Houbern-Weyl : Methoden der organischen Chemie, Bd. XIII/I., S. 134-159 bzw. 389-405 (1970) erhältlich.

Die Hydroxyketone der allgemeinen Formel (II) sind zum Beispiel durch die Fries-Reaktion synthetisierbar (A.H. Blatt : The Fries reaction in Organic reactions, Vol. Ip, p. 342). Die Ausgangsverbindungen der allgemeinen Formel (III) sind zum Beispiel durch die Umsetzung von 4-Benzyloxypropiophenon mit einem entsprechend substituierten Phenylmagnesiumhalogenid zugänglich ; dies ist in dem oben zitierten Buch von M.S. Kharash et al. auf den Seiten 138-143 beschrieben.

Bei der Durchführung des Verfahrens setzt man das 4-Hydroxypropiophenon mit vorzugsweise mindestens der doppelten molaren Menge der metallorganischen Verbindung der allgemeinen Formel (I) in einem wasserfreien inerten organischen Lösungsmittel, zweckmäßig unter Schutzgas, um. Als metallorganische Verbindung setzt man zweckmäßig das entsprechend substituierte Phenyllithium oder die entsprechend substituierten Phenylmagnesiumhalogenide, insbesondere das Chlorid oder Bromid, ein. Die Umsetzung wird in einem aprotischen organischen Lösungsmittel, zum Beispiel Hexamethylphosphoramid, Dimethylsulfoxyd, aliphatischen oder cycloaliphatischen Äthern, wie Diäthyläther, Dibutyläther, Äthylenglykoldimethyläther, Dioxan oder Tetrahydrofuran, in aliphatischen oder aromatischen Kohlenwasserstoffen, zum Beispiel Ligroin, Benzol, Toluol, Xylol, oder in Gemischen der aufgeführten Lösungsmittel vorgenommen. Als Schutzgas wird zum Beispiel Stickstoff oder Argon verwendet. Die Reaktionstemperatur liegt zwischen —70 °C und dem Siedepunkt des Lösungsmittels und beträgt vorzugsweise —40 bis 100 °C. Das Reaktionsgemisch wird in an sich bekannter Weise aufgearbeitet, zum Beispiel, indem man die Grignard-Verbindung durch Eingießen des Reaktionsgemisches in verdünnte organische oder anorganische Säuren, zum Beispiel Schwefelsäure, Salzsäure, Essigsäure, oder vorzugsweise in die wäßrige Lösung von Ammoniumchlorid zersetzt und die gebildete Verbindung isoliert. Das Produkt kann durch Chromatographie oder durch Umkristallisieren gereinigt werden.

Das Herstellungsverfahren wird an Hand des folgenden Beispiels näher erläutert.

## Beispiel 1

3-Trifluormethyl-4'-hydroxy-α-äthylbenzhydrol

Zu der mit Tetrahydrofuran bereiteten Lösung des aus 11 g metallischem Magnesium und 101,3 g 3-(Trifluormethyl)-brombenzol hergestellten Grignard-Reagens wird bei 66-68 °C die Lösung von 22,5 g 4-Hydroxypropiophenon in 405 ml Tetrahydrofuran gegeben. Nach Beendigung der Zugabe wird das Gemisch eine Stunde lang gekocht. Nachdem das Ende der Reaktion dünnschichtchromatographisch festgestellt wurde, wird das Gemisch auf 0 °C gekühlt und in eine kältemischung aus Eis und Eisessig gegossen. Die organische Phase wird mit gesättigter Kochsalzlösung neutral gewaschen, über Natriumsulfat getrocknet und dann das Lösungsmittel unter vermindertem Druck abdestilliert. Das Rohprodukt wird mit Aktivkohle geklärt und dann aus einem Gemisch von Äthylacetat und n-Hexan umkristallisiert. Man erhält 30,5 g eines kristallinen Produktes, das bei 102,5-103 °C schmilzt.

Elementaranalyse für die Summenformel $C_{16}H_{15}F_3O_2$

| | | | |
|---|---|---|---|
| berechnet : | C 64,86 | H 5,10 | F 19,24 % |
| gefunden : | C 64,89 | H 5,16 | F 18,83 % |

## Beispiel 2

Aus den im Patentanspruch genannten Verbindungen können zum Beispiel die folgenden Arzneimittelpräparate bereitet werden.

Tabletten

Zusammensetzung einer Tablette

| | |
|---|---|
| Wirkstoff | 100,0 mg |
| Lactose | 184,0 mg |
| Kartoffelstärke | 80,0 mg |
| Polyvinylpyrrolidon | 8,0 mg |
| Talk | 12,0 mg |
| Magnesiumstearat | 2,0 mg |
| Aerosil® (kolloidales $SiO_2$) | 2,0 mg |
| Ultraamylopektin | 12,0 mg |

Durch Naßgranulieren und Pressen werden aus den angegebenen Stoffen Tabletten von 400 mg Gewicht hergestellt. Wirkstoff : 3-Trifluormethyl-4'-hydroxy-α-äthylbenzhydrol.

Dragees

Die auf die beschriebene Weise erhaltenen Tabletten werden in an sich bekannter Weise mit einem aus Zucker und Talk bestehenden Überzug versehen und dann mit einem Gemisch aus Bienenwachs und Carnaubawachs poliert. Gewicht eines Dragees : 500,0 mg.

Kapseln

Zusammensetzung der Füllung einer Kapsel

| | |
|---|---|
| Wirkstoff | 50,0 mg |
| Lactose | 100,0 mg |
| Talk | 2,0 mg |
| Kartoffelstärke | 30,0 mg |
| Mikrokristalline Cellulose | 8,0 mg |

Der Wirkstoff wird gründlich mit den Hilfsstoffen vermischt, das Gemisch durch ein Sieb der Maschenweite 0,32 mm gesiebt und dann in Hartgelatinekapseln (Größe 4) gefüllt. Wirkstoff : 3-Trifluormethyl-4'-hydroxy-α-äthylbenzhydrol.

Suppositorien

Zusammensetzung eines Suppositoriums

| | |
|---|---|
| Wirkstoff | 100,0 mg |
| Lactose | 200,0 mg |
| Suppositoriengrundmasse (z. B. Witepsol® H) | 1 700,0 mg |

Die Grundmasse wird geschmolzen und die Schmelze auf 35 °C gekühlt. Der Wirkstoff wird gründlich mit der Lactose vermischt und das Gemisch in einem Homogenisator in der Schmelze homogenisiert. Das erhaltene Gemisch wird in gekühlte Suppositorienformen gegossen. Gewicht eines Suppositoriums : 2 000 mg.
Wirkstoff : 3-Trifluormethyl-4'-hydroxy-α-äthylbenzhydrol.

Suspension

100 ml Suspension enthalten :

| | |
|---|---|
| Wirkstoff | 1,0 g |
| Natriumhydroxyd | 0,26 g |
| Citronensäure | 0,30 g |
| Nipagin® (Natriumsalz des 4-Hydroxybenzoesäuremethylesters) | 0,10 g |
| Carbopol® 940 (Polyacrylsäure) | 0,30 g |
| Äthanol (96 %ig) | 1,00 g |
| Himbeeraroma | 0,60 g |
| Sorbit (70 %ige wäßrige Lösung) | 71,00 g |
| dest. Wasser zum Auffüllen auf | 100,00 ml |

Das Nipagin® und die Citronensäure werden in 20 ml destilliertem Wasser gelöst. Zu der Lösung gibt man in kleinen Portionen unter intensivem Rühren das Carbopol® und läßt die Lösung dann 10-12 Stunden lang stehen. Anschließend werden die mit 1 ml destilliertem Wasser bereitete Lösung des Natriumhydroxyds, dann die wäßrige Lösung des Sorbits und schließlich die äthanolische Lösung des Himbeeraromas unter Rühren zugegeben. Zu der so bereiteten Trägersubstanz gibt man in kleinen Portionen den Wirkstoff und homogenisiert das Ganze mit einem Mischer. Die Suspension wird mit destilliertem Wasser auf 100 ml aufgefüllt und in einer Kolloidmühle homogenisiert.
Wirkstoff : 3-Trifluormethyl-4'-hydroxy-α-äthylbenzhydrol.

**Patentanspruch** (für die Vertragsstaaten : BE, CH, DE, FR, GB, IT, LI, LU, NL, SE)

Substituierte 4'-Hydroxy-α-äthylbenzhydrole der allgemeinen Formel

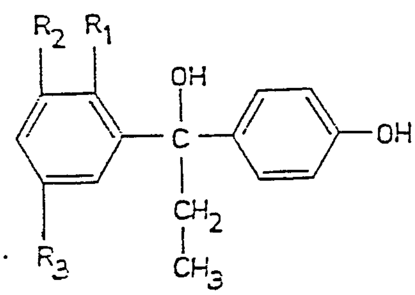

0,

worin
$R_1$ und $R_3$ für Methylreste stehen und
$R_2$ Wasserstoff bedeutet, oder
$R_1$ und $R_3$ für Wasserstoff stehen und
$R_2$ einen Trifluormethylrest bedeutet,
zur Verwendung als Arzneimittel zur Behandlung von akuten Alkoholvergiftungen.

**Patentansprüche** (für den Vertragsstaat AT)

1. Verwendung von substituierten 4'-Hydroxy-α-ethylbenzhydrolen der allgemeinen Formel

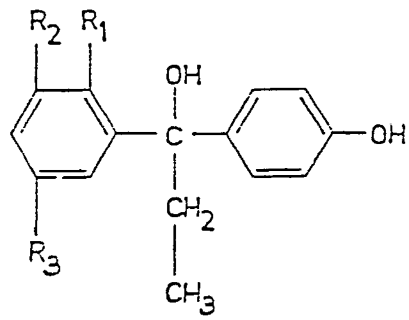

0,

worin
$R_1$ und $R_3$ für Methylreste stehen und
$R_2$ Wasserstoff bedeutet, oder
$R_1$ und $R_3$ für Wasserstoff stehen und
$R_2$ einen Trifluormethylrest bedeutet,
als Wirkstoff zur Herstellung von Arzneimitteln zur Behandlung von akuten Alkoholvergiftungen.

2. Verfahren zur Herstellung von Arzneimitteln zur Behandlung von akuten Alkoholvergiftungen, dadurch gekennzeichnet, daß 4'-Hydroxy-α-ethylbenzhydrole der allgemeinen Formel

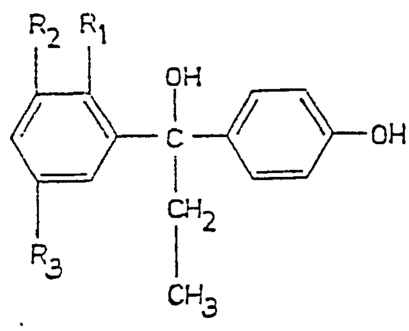

0,

worin
$R_1$ und $R_3$ für Methylreste stehen und
$R_2$ Wasserstoff bedeutet, oder
$R_1$ und $R_3$ für Wasserstoff stehen und
$R_2$ einen Trifluormethylrest bedeutet,
mit üblichen Träger- und/oder Hilfsstoffen vermischt und die Mischungen zu insbesondere oralen, rektalen und/oder parenteralen Darreichungsformen verarbeitet werden.

**Claim** (for the Contracting States : BE, CH, DE, FR, GB, IT, LI, LU, NL, SE)

Substituted 4'-hydroxy-α-ethyl-benzhydrols of the general formula

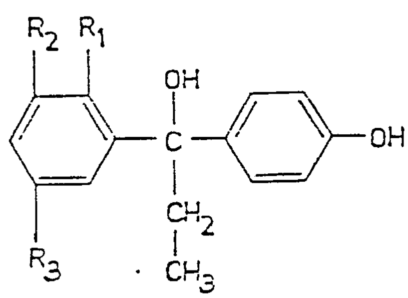

0,

wherein
$R_1$ and $R_3$ are standing for methyl radicals, and
$R_2$ represents hydrogen, or
$R_1$ and $R_3$ are standing for hydrogen, and
$R_2$ represents a trifluoromethyl radical,
for use as a medicament for the treatment of acute alcohol poisoning.

**Claims** (for the Contracting State AT)

1. The use of substituted 4'-hydroxy-α-ethyl-benzhydrols of the general formula

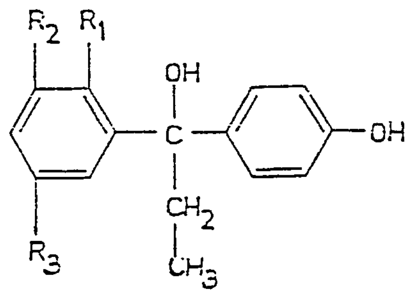

0,

wherein
$R_1$ and $R_3$ are standing for methyl radicals, and
$R_2$ represents hydrogen, or
$R_1$ and $R_3$ are standing for hydrogen, and
$R_2$ represents a trifluoromethyl radical,
as active ingredients for the preparation of pharmaceuticals for the treatment of acute alcohol toxications.

2. A process for preparing pharmaceuticals for the treatment of acute alcohol toxications, characterized in that 4'-hydroxy-α-ethyl-benzhydroles of the general formula

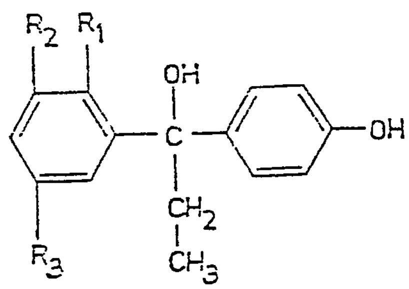

0,

wherein
$R_1$ and $R_3$ are standing for methyl radicals, and
$R_2$ represents hydrogen, or
$R_1$ and $R_3$ are standing for hydrogen, and
$R_2$ represents a trifluoromethyl radical,
are mixed with conventional carriers and/or adjuvants, and the mixtures are processed to especially oral, rectal and/or parenteral application forms.

**Revendication** (pour les Etats contractants : BE, CH, DE, FR, GB, IT, LI, LU, NL, SE)

4'-Hydroxy-α-éthylbenzhydrols substitués répondant à la formule générale

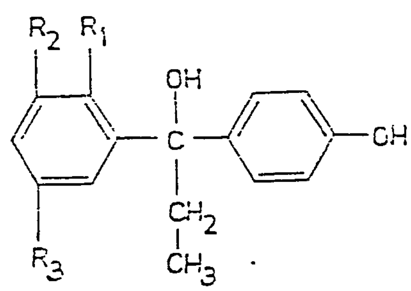

0,

dans laquelle

$R_1$ et $R_3$ représentent des restes méthyle, et

$R_2$ représente l'hydrogène, ou

$R_1$ et $R_3$ représentent l'hydrogène, et

$R_2$ représente un reste trifluorométhyle

à utiliser comme médicaments pour le traitement d'intoxications alcooliques aiguës.

**Revendications** (pour l'Etat contractant AT)

1. L'utilisation de 4'-hydroxy-α-éthylbenzhydroles substitués répondant à la formule générale

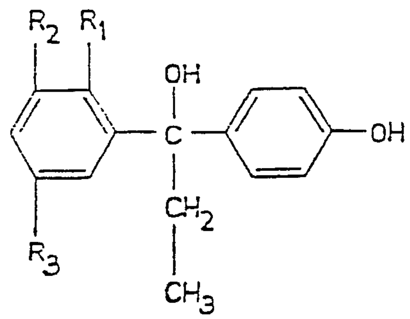

0,

dans laquelle

$R_1$ et $R_3$ représentent des restes méthyle, et

$R_2$ représente l'hydrogène, ou

$R_1$ et $R_3$ représentent l'hydrogène, et

$R_2$ représente un reste trifluorométhyle

comme ingrédient actif pour la préparation des médicaments pour le traitement des intoxications alcooliques aiguës.

2. Procédé pour la préparation des médicaments pour le traitement des intoxications alcooliques aiguës, caractérisé en ce que 4'-Hydroxy-α-éthylbenzhydroles répondant à la formule générale

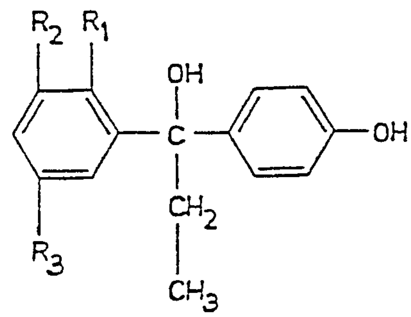

0,

dans laquelle

$R_1$ et $R_3$ représentent des restes méthyle, et

$R_2$ représente l'hydrogène, ou

$R_1$ et $R_3$ représentent l'hydrogène, et

$R_2$ représente un reste trifluorométhyle

sont mélangés avec des supports et/ou les adjoints conventionnels et les mixtures sont spécialement utilisés à une forme de présentation orale, rectale et/ou parentérale.